(19) Europäisches Patentamt
European Patent Office
Office européen des brevets

(11) **EP 3 773 419 B1**

(12) **EUROPÄISCHE PATENTSCHRIFT**

(45) Veröffentlichungstag und Bekanntmachung des Hinweises auf die Patenterteilung:
**08.05.2024 Patentblatt 2024/19**

(21) Anmeldenummer: **18743717.3**

(22) Anmeldetag: **12.07.2018**

(51) Internationale Patentklassifikation (IPC):
**A61K 6/889** *(2020.01)*

(52) Gemeinsame Patentklassifikation (CPC):
**A61K 6/889**

(86) Internationale Anmeldenummer:
**PCT/EP2018/068948**

(87) Internationale Veröffentlichungsnummer:
**WO 2019/185175 (03.10.2019 Gazette 2019/40)**

(54) **WASSERHÄRTENDER DENTALZEMENT, VERFAHREN UND KIT ZUM HERSTELLEN EINES SOLCHEN UND DESSEN VERWENDUNG**

WATER-HARDENING DENTAL CEMENT, METHOD AND KIT FOR MANUFACTURING SUCH AND ITS USE

CIMENT DENTAIRE DURCISSANT L'EAU, PROCÉDÉ ET KIT DE FABRICATION ET SON UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.03.2018 DE 102018204655**

(43) Veröffentlichungstag der Anmeldung:
**17.02.2021 Patentblatt 2021/07**

(60) Teilanmeldung:
**24156250.3 / 4 360 610**

(73) Patentinhaber: **Mühlbauer Technology GmbH 22547 Hamburg (DE)**

(72) Erfinder:
• **BÖTTCHER, Henrik**
  **21255 Tostedt (DE)**
• **NEFFGEN, Stephan**
  **25421 Pinneberg (DE)**
• **MÜLLER, Holger**
  **25421 Pinneberg (DE)**

(74) Vertreter: **Glawe, Delfs, Moll Partnerschaft mbB von Patent- und Rechtsanwälten Postfach 13 03 91 20103 Hamburg (DE)**

(56) Entgegenhaltungen:
| | |
|---|---|
| EP-A1- 1 767 184 | EP-A1- 2 316 407 |
| WO-A1-2010/102786 | WO-A2-02/092022 |
| DE-A1-102006 019 092 | JP-A- 2009 184 971 |

**Beschreibung**

Die Erfindung betrifft einen wasserhärtenden Dentalzement, ein Verfahren zum Herstellen eines wasserhärtenden Dentalzements, ein Kit zum Herstellen eines wasserhärtenden Dentalzements sowie dessen Verwendung als zahnärztliches Füllungs- und/oder Befestigungsmaterial.

Bei wasserhärtendem Dentalzement handelt es sich chemisch betrachtet um Substanzen, die beim Vermischen von Pulver und Flüssigkeit über eine Säure-Base Reaktion abbinden. Hierfür enthalten die Dentalzemente mindestens drei Bestandteile: ein säurereaktives Pulver als Base, eine Säure und Wasser.

Bei klassischen Glasionomerzementen (KGIZ) handelt es sich um wasserhärtende Zemente, die im Wesentlichen die folgenden drei Bestandteile umfassen: säurereaktives Glaspulver, eine Polyalkensäure und Wasser. Das Pulver enthält beispielsweise ein säurereaktives Fluoralumosilikatglas. Die Flüssigkeit enthält Wasser. Die Säure ist meist eine wasserlösliche Polyalkensäure, die entweder im Pulver oder in der Flüssigkeit enthalten sein kann.

Ein im Stand der Technik bekannter klassischer Glasionomerzement für Füllungen (Kavitätenklassen III und V, Wurzelkaries, Tunnelpräparationen, Milchzahnkavitäten und Unterfüllungen) wird beispielsweise unter dem Namen Alpha® Fil (DMG) vertrieben. Weitere bekannte Produkte sind GC Fuji IX® und Ketac® Fil.

Der kritische Spannungsintensitätsfaktor $K_{Ic}$ ist ein Maß für die Riss- bzw. Bruchzähigkeit eines Materials. Das Problem bekannter Zemente ist, dass sie nur eine mangelnde Riss- und Bruchzähigkeit aufweisen. Die vorliegende Erfindung soll dieses Problem lösen.

Im Stand der Technik wird versucht, die Bruchzähigkeit wasserhaltiger Dentalzemente durch den Zusatz polymerisierbarer Komponenten, insbesondere durch den Zusatz von (Meth)acrylatgruppen aufweisenden Monomeren, Polymeren und/oder Füllstoffen zu verbessern. Solche polymerisierbaren Zemente sind jedoch mit Nachteilen gegenüber den klassischen Glasionomerzementen verbunden, beispielsweise verringerte Biokompatibilität.

JP 2001354509 beschreibt eine Pulvermischung für Glasionomerzemente mit verbesserter Mechanik, insbesondere Bruchzähigkeit, die Fasern auf Basis von Apatit bzw. Fluorapatit enthält. Die Fasern bewirken eine anisotrope Verstärkung. Ein Nachteil der verwendeten Fasern ist allerdings, dass diese eine unerwünscht raue Oberfläche erzeugen können.

US 6 860 932 beschreibt eine Verbesserung der Bruchzähigkeit durch $TiO_2$- oder $Al_2O_3$-Partikel. Die beschriebenen Metalloxide haben einen hohen Brechungsindex und erhöhen damit die Opazität des Glasionomerzementen, was eine zahnähnliche Einfärbung erschwert.

WO 2017083039 A1 offenbart ein Kit zum Herstellen eines Glasionomerzementes, welches als einen Bestandteil Aluminium- oder Siliciumdioxid-basierte Partikel umfasst.

EP 2 316 407 A1 beschreibt eine dentale Glasionomerzementzusammensetzung mit dispergierten Nanopartikeln, die gepfropfte lineare oder verzweigte Polymerketten mit Säuregruppen enthalten. Damit sollen Biegefestigkeit und Bruchzähigkeit des gehärteten Zements erhöht werden.

Der Erfindung liegt die Aufgabe zu Grunde, einen wasserhärtenden Dentalzement zu schaffen, der eine verbesserte Riss- und Bruchzähigkeit aufweist, ohne dabei nennenswert sonstige vorteilhafte Eigenschaften einzubüßen.

Gelöst wird diese Aufgabe durch einen wasserhärtenden Dentalzement gemäß dem Hauptanspruch. Die Erfindung betrifft somit insbesondere einen wasserhärtenden Dentalzement umfassend ein säurereaktives Pulver, eine mehrprotonige Säure, Wasser und dispergierte Polymerpartikel, wobei die Polymerpartikeln Polyurethanpartikeln sind.

Weitere vorteilhafte Ausführungsformen finden sich in den Unteransprüchen.

Zunächst seien einige im Rahmen der Erfindung verwendete Begriffe erläutert.

Der kritische Spannungsintensitätsfaktor $K_{Ic}$ ist ein Maß für die Riss- bzw. Bruchzähigkeit eines Materials. Der Spannungsintensitätsfaktor K ist ein Maß für die Intensität des Spannungsfeldes in der Nähe einer Rissspitze. Derjenige Spannungsintensitätsfaktor, bei dem es schließlich zum Bruch kommt, ist der kritische Spannungsintensitätsfaktor. Dieser Werkstoffkennwert wird auch als Riss- oder Bruchzähigkeit bezeichnet. Der kritische Spannungsintensitätsfaktor $K_{Ic}$ charakterisiert Riss- bzw. Bruchzähigkeit bei Rissöffnung senkrecht zur Rissfläche. Dieser Rissöffnungsmodus hat die größte Bedeutung in der Praxis.

Unter einem säurereaktiven Pulver wird in Rahmen der Erfindung ein Pulver verstanden, dass mit einer mehrprotonigen Säure über eine Säure-Base-Reaktion reagiert. Geeignete säurereaktive Pulver sind im Stand der Technik bekannt und werden im Folgenden näher erläutert.

Unter einer mehrprotonigen Säure wird im Stand der Technik und im Rahmen der Erfindung eine Säure verstanden, die mehrere Protonen aufweist und bei der die Protonenabgabe schrittweise über mehrere Dissoziationsstufen erfolgt. Für die Erfindung geeignete mehrprotonige Säuren werden im Folgenden näher erläutert.

Kern der Erfindung ist es, einen wasserhärtenden Dentalzement, bevorzugt einen klassischen Glasionomerzement, mit darin dispergierten Polymerpartikeln zu versehen, wodurch eine zum Bruch führende Rissausbreitung im Zement erschwert wird. Somit erfolgt eine Verbesserung der Riss- und Bruchzähigkeit des wasserhärtenden Zements in einfacher Weise und ohne die Verschlechterung anderer Eigenschaften. Die Erhöhung der Riss- und Bruchzähigkeit bzw. des kritischen Spannungsintensitätsfaktors $K_{Ic}$ des wasserhärtenden Zements hat wiederum eine Erhöhung der Haltbarkeit einer Füllung zur Folge und kann eine Indikationserweiterung, insbesondere auf kaulasttragende Füllungen ermöglichen.

Somit wird im Rahmen der Erfindung unter Erhalt oder Verbesserung der sonstigen vorteilhaften Eigenschaften der wasserhärtenden Dentalzemente die Risszähigkeit bzw. der kritische Spannungsintensitätsfaktor $K_{Ic}$ verbessert. Beispiele sonstiger zu beachtender Eigenschaften von wasserhärtendem Dentalzement, insbesondere klassischem Glasionomerzement, sind Konsistenz/Modellierbarkeit, Verarbeitungszeit, Abbindezeit (1,5-6 min), Druckfestigkeit (>100, >180 MPa), Biegefestigkeit (>25 MPa), Abrasion/Säureerosion (<0,17 mm), optische Eigenschaften (Opazität KGIZ C0,70 von 0,35-0,90), Röntgensichtbarkeit ($\triangleq$ %Al), Farbton und Farbbeständigkeit, Eigenhaftung/Feuchtigkeitstoleranz, Biokompatibilität/Empfindlichkeiten, Fluoridfreisetzung und Expansionsverhalten. Die angegebenen Mindestanforderungen der klassischen Glasionomerzement sind der ISO9917 entnommen.

Weitere Vorteile der Erfindung sind, dass die erfindungsgemäßen Polymerpartikel toxikologisch unbedenklich sind und nur einen sehr geringen Gewichtsanteil im Zement ausmachen. Als Hauptkomponenten im Dentalzement können weiterhin kommerziell erhältliche und klinisch bewährte Zementbestandteile gewählt werden. Insbesondere kann auch auf den Zusatz von polymerisierbaren Verbindungen, wie (Meth)acrylaten, verzichtet werden.

Im Rahmen der Erfindung ist es bevorzugt, dass der wasserhärtende Dentalzement ein Glasionomerzement, bevorzugter ein klassischer Glasionomerzement (KGIZ) ist.

Klassische oder konventionelle Glasionomerzemente sind im Stand der Technik bekannt, sie härten durch eine Säure-Base-Reaktion aus. Neben den klassischen Glasionomerzementen existieren beispielsweise noch sogenannte metallverstärkte Glasionomerzemente oder Cermet-Zemente (z.B. Ketac® Silver und Alpha® Silver) und kunststoffmodifizierte Glasionomerzemente (z.B. Photac® Fil Quick, Vitremer® Fuji® II LC). Ein Vorteil der klassischen Glasionomerzemente ist beispielsweise ihre gute chemische Haftung an Zahnhartsubstanzen.

Polymerpartikel

Geeignete Polymerpartikel bilden in Wasser bzw. in einer wässrigen Phase eine Polymerdispersion.

Bevorzugt weisen die Polymerpartikel an ihrer Oberfläche ionische Gruppen und/oder in wässriger Phase sterisch stabilisierende Gruppen auf.

Unter ionischen Gruppen werden hier alle elektrostatisch stabilisierenden Gruppen verstanden, insbesondere anionische und kationische Gruppen.

Unter sterisch stabilisierenden Gruppen werden hier polymere oder oligomere Kettenelemente verstanden, die mindestens in Wasser mindestens zum Teil löslich sind. Sie stabilisieren die dispergierten Partikel aufgrund entropischer Effekte nach dem Mechanismus der sterischen Stabilisierung (T.S. Tadros, Interfacial Phenomena and Colloid Stability, Vol. 1 Basic Principles, De Gruyter 2015, 209 ff.) in wässrigen Medien gegen Agglomeration und Koagulation. Allgemein als polymere oder oligomere Kettenelemente geeignete und bevorzugte Polymerketten sind bei der näheren Beschreibung einzelner Polymerpartikel beschrieben.

Bevorzugt sind die ionisch und/oder sterisch stabilisierenden Gruppen überwiegend kovalent an das Polymer der Partikel gebunden.

Bevorzugt sind die Polymerpartikel kugelförmig.

Bevorzugt werden die Polymerpartikel als Primär- und/oder Sekundärdispersionen hergestellt.

Weiter ist es bevorzugt, dass eine mittlere Partikelgröße der Polymerpartikel kleiner als etwa 1 $\mu$m, bevorzugter zwischen 5 bis 500 nm, noch bevorzugter zwischen 5 bis 100 nm ist. Die Partikelgröße ist bestimmbar durch dynamische Lichtstreuung (z.B. Zeta-Sizer Nano-zs, Fa. Malvern) der wässrigen Dispersionen der Polymerpartikel. Die Polymerpartikel liegen in aller Regel bereits nach Herstellung als wässrige Dispersionen vor, die nach dem Fachmann bekannten Verfahren für die Lichtstreumessung aufgearbeitet werden. Sofern die Polymerpartikel als trockener Feststoff, bspw. als Pulver vorliegen, werden sie vor der Lichtstreumessung in eine wässrige Dispersion überführt, bspw. unter Anwendung von Rühr-Dispergier- und/oder Ultraschalleinrichtungen (z.B. Ultraschall-Homogenisatoren wie Sonopuls 4200, Fa. Bandelin). Weiter ist es bevorzugt, dass die Polymerpartikel für die Herstellung des wasserhärtenden Dentalzements, d.h. vor dem Aushärten des wasserhärtenden Zements, als wasserhaltige Dispersion vorliegen.

Die erfindungsgemäßen dispergierten Polymerpartikel sind insbesondere dadurch charakterisiert, dass sie mit Wasser als Dispersionsmedium eine stabile disperse Phase bilden. Stabil meint dabei, dass die dispergierten Polymerpartikel für mindestens eine Stunde und bevorzugt über eine Haltbarkeit eines Verkaufsproduktes, bevorzugt mindestens 6 Monate in Wasser als Dispersionsmedium im Wesentlichen dispergiert bleiben und nicht sedimentieren und/oder agglomerieren und/oder aggregieren.

Zudem ist es bevorzugt, dass ein Anteil dispergierter Polymerpartikel im Dentalzement mindestens 0,005 Gew.-%, bevorzugter mindestens 0,01 Gew.-%, bezogen auf die Gesamtzusammensetzung des Dentalzements vor der Aushärtung beträgt.

Weiter ist es bevorzugt, dass ein Anteil dispergierter Polymerpartikel im Dentalzement höchstens 10 Gew.-%, bevorzugter höchstens 3 Gew.-%, noch bevorzugter höchstens 1 Gew.-%, weiter bevorzugt höchstens 0,5 Gew.-%, am meisten bevorzugt höchstens 0,3 Gew.-%, bezogen auf die Gesamtzusammensetzung des Dentalzements vor der Aushärtung, beträgt.

Bevorzugter ist es ferner, dass ein Anteil dispergierter Polymerpartikel im Dentalzement 0,005 bis 5 Gew.-%, bevorzugter 0,005 bis 3 Gew.-%, noch bevorzugter 0,01 bis 1 Gew.-%, weiter bevorzugt 0,01 bis 0,5 Gew.-%, am meisten bevorzugt 0,01 bis 0,3 Gew.-%, bezogen auf die Gesamtzusammensetzung des Dentalzements vor der Aushärtung, beträgt.

Bevorzugte Polymerpartikel sind Polymerpartikel, die mittels Emulsionspolymerisation hergestellt sind.

Bevorzugte Polymerpartikel enthalten Polymerketten, die durch ggf. substituierte Homoketten (s. H.G. Elias, Makromoleküle, Band 1: Chemische Struktur und Synthesen, 6. Auflage, Wiley VCH) aufgebaut sind.

Polymerpartikel auf der Basis von Homoketten können in einer spezifischen Ausführungsform vernetzt sein.

Vernetzte Strukturen entstehen in einer Herstellungsvariante durch die Copolymerisation von Monomeren mit einer copolymerisierbaren Gruppe im

Emulsionspolymerisationsverfahren mit Monomeren mit mehr als einer copolymerisierbaren Gruppe.

Weitere geeignete Polymerpartikel zum Einsatz in den erfindungsgemäßen Dentalmaterialien lassen sich durch Miniemulsionspolymerisation herstellen. In diesem Verfahren lassen sich sowohl nach radikalischem Mechanismus als auch durch Polyaddition geeignete Partikel herstellen (K. Landfester, F. Tiarks, H.-P. Hentze, M. Antonietti, Macromol. Chem. Phys. 201, (2000) 1-5; K. Landfester, Macromol. Rapid Commun. 22, (2001) 896).

Bevorzugte Polymerpartikel sind Kern-Schale-Partikel, bei denen sich ein innerer Teil des Partikels stofflich und potentiell auch in anderen Eigenschaften, wie bspw. dem E-Modul, von einem äußeren Teil unterscheidet. Unter Schale sei in diesem Zusammenhang nicht eine Schicht von sterisch oder elektrostatisch stabilisierenden Gruppen verstanden. Bevorzugte Kern-Schale-Partikel können einen weicheren Kern und eine härtere Schale aufweisen. In einer anderen Ausführungsform besitzen sie einen härteren Kern und eine weichere Schale. Die Kern-Schale-Partikel enthalten bevorzugt im Wesentlichen Homoketten. Die Kern-Schale-Partikel werden bevorzugt über zweistufige Emulsionspolymerisationsverfahren hergestellt. Die Erfindungsgemäße Kern-Schale Partikel sind PU-Partikel. In einer bevorzugten Ausführungsform weisen die Kern-Schale Partikel einen Polyurethankern und eine Schale aus Polymerketten, die durch ggf. substituierte Homoketten aufgebaut sind

Die erfindungsgemäßen Polymerpartikel sind Polyurethanpartikel (PU-Partikel).

Bevorzugte PU-Partikel sind solche, die hergestellt sind aus:

a) mindestens einem Polyisocyanat,
b) optional mindestens einer Verbindung, die im Sinne einer Isocyanatreaktion mono- oder difunktionell ist, insbesondere einem Polyol A
c) mindestens einer weiteren Verbindung, die im Sinne einer Isocyanatreaktion mono- oder difunktionell ist und die zusätzlich mindestens eine ionische Gruppe und/oder mindestens eine in eine ionische Gruppe umwandelbare funktionelle Gruppe und/oder mindestens eine sterisch stabilisierende Gruppe umfasst, und
d) optional mindestens einer weiteren Verbindung, die im Sinne einer Isocyanatreaktion mono- oder di- oder höherfunktionell ist und ausgewählt ist aus der Gruppe Polyol B, Kettenverlängerer und Vernetzer, wobei mindestens eine der beiden Komponenten b) oder d) vorhanden sein muss.

Bevorzugte PU-Partikel sind solche, die hergestellt sind aus:

a) mindestens einem Polyisocyanat,
b) mindestens einem Polyol A,
c) mindestens einer weiteren Verbindung, die im Sinne einer Isocyanatreaktion mono- oder difunktionell ist und die zusätzlich mindestens eine ionische Gruppe und/oder mindestens eine in eine ionische Gruppe umwandelbare funktionelle Gruppe und/oder mindestens eine sterisch stabilisierende Gruppe umfasst, und
d) optional mindestens einem Polyol B und/oder einem Kettenverlängerer und/oder einem Vernetzer.

Verbindungen, die im Sinne einer Isocyanat-Reaktion mono- oder difunktionell sind, sind solche, die Gruppen enthalten, die mit Isocyanat reagieren können. Dabei handelt es sich bevorzugt um funktionelle Gruppen, die aktiven Wasserstoff enthalten, wie beispielsweise OH, $NH_2$, NH, $NHNH_2$, SH und ähnliche. Solche Gruppen sind dem Fachmann bekannt und beispielsweise in D. Randall, S. Lee (eds.), The Polyurethanes Book, John Wiley & Sons, Ltd, 2002 beschrieben. Die Auswahl der Verbindungen erfolgt vom Fachmann je nach gewünschten Eigenschaften.

Geeignete Polyisocyanate weisen mindestens zwei Isocyanatgruppen auf. Bevorzugt weist das mindestens eine Polyisocyanat genau zwei Isocyanatgruppen auf. Bevorzugt ist aber auch, dass das mindestens eine Polyisocyanat ein höherfunktionelles Polyisocyanat mit mehr als zwei Isocyanatgruppen ist. Bevorzugt ist das mindestens eine Polyisocyanat eine aromatische oder aliphatische, insbesondere eine acyclische oder cyclische, Verbindung. Besonderes bevorzugt ist es, wenn das mindestens eine Polyisocyanat eine aliphatische, noch bevorzugter ein cycloaliphatische, Verbindung ist. Bevorzugt handelt es sich bei dem mindestens einem Polyisiocyanat um ein modifiziertes oder nicht modifiziertes. Geeignete modifizierte Polyisocyanate sind beispielsweise solche, die Carbodiimidgruppen, Allophanatgruppen, Isocyanuratgruppen, Urethangruppen und/oder Biuretgruppen enthalten. Bevorzugt ist es auch, dass eine Kombination aus modifizierten und nicht modifizierten Polyisocyanten verwendet wird.

Bevorzugte Polyisocyanate sind beispielsweise Tetramethylendiisocyanat, Hexamethylendiisocyanat, Isophorondiisocyanat, 1,4-Diisocyanatocyclohexan, 1,3-Bis(isocyanatomethyl)cyclohexan, 4,4'-Diisocyanatodicyclohexylmethan, 1,4-Phenylendiisocyanat, 2,6-Toluoldiisocyanat, 2,4-Toluoldiisocyanat, m-Xylendiisocyanat, 1,3-Bis(1-isocyanato-1-methylethyl)benzol, 2,4'-Diphenylmethandiisocyanat und 4,4'-Diphenylmethandiisocyanat. Besonders bevorzugte Polyisocycanate sind ausgewählt aus aliphatischen und cycloaliphatischen Polyisocyanaten.

Das Polyol A weist bevorzugt eine Hydroxygruppe und mindestens eine weitere Hydroxy- oder Aminogruppe auf.

In einer weiteren Ausführungsform weist das Polyol A zwei oder mehr Aminogruppen in bevorzugt terminaler Position auf, wie $\alpha,\omega$-Diaminopolyether, bspw. die von der Huntsman Corporation vertriebenen Jeffamin®-Polyetheramin-Typen.

Bevorzugt weist das Polyol A ein Molekulargewicht zwischen 500 und 6000 g/mol, bevorzugter zwischen 500 und 2000 g/mol, auf. Geeignetes Polyol A kann bevorzugt linear oder verzweigt sein. Geeignete Polyole A sind beispielsweise Polyetherpolyole, Polyesterpolyole, Polyesteramidpolyole, Polycarbonatpolyole, Polyolefinpolyole, Polysiloxanpolyole und Poly(meth)acrylatpolyole, noch bevorzugter jeweils mit endständigen Hydroxygruppen. Besonders bevorzugt sind Polyetherpolyole. Am meisten bevorzugt ist Polytetrahydrofuran 1000.

Polyetherpolyole sind bevorzugt das Reaktionsprodukt der Polymerisation von cyclischen, organischen Oxiden wie beispielsweise Ethylenoxid, Propylenoxid oder Tetrahydrofuran durch polyfunktionelle Initiatoren wie Wasser, Ethylenglycol, Propylenglycol, Diethylenglycol, Cyclohexandimethanol, Glycerin, Trimethylolpropan, Pentaerythrit. Es können auch Gemische von cyclischen, organischen Oxiden verwendet werden. Die Polymerisation von verschiedenen cyclischen, organischen Oxiden kann gleichzeitig unter Bildung statistischer Copolymere erfolgen, oder die Zugabe kann nacheinander erfolgen unter Bildung von Blockcopolymeren.

Polyesterpolyole werden bevorzugt durch Umsetzung von mindestens zweiwertigen Alkoholen mit mindestens zweiwertigen Carbonsäuren erhalten. Die Carbonsäuren können dabei aliphatisch, cycloaliphatisch, heterocyclisch, araliphatisch oder aromatisch sein. Neben den freien Carbonsäuren können auch entsprechende Carbonsäureanhyride oder die Methyl- oder Ethylester der entsprechenden Carbonsäuren eingesetzt werden. Es können auch Gemische unterschiedlicher, mehrwertiger Carbonsäuren und deren Derivate mit Gemischen, unterschiedlicher mehrwertiger Alkohole umgesetzt werden. Geeignete Carbonsäuren, Carbonsäureanhydride und Carbonsäureester sind beispielsweise Bernsteinsäure, Bernsteinsäureanhydrid, Bernsteinsäuredimethylester, Adipinsäure, Adipinsäuredimethylester, Glutarsäure, Glutarsäuredimethylester, 1,4-Cyclohexandicarbonsäure, Maleinsäure, Maleinsäureanhydrid, Fumarsäure, Phthalsäure, Isophthalsäure, Phthalsäureanhydrid, Tetrahydrophthalsäure, Hexahydrophthalsäure oder Terephthalsäuredimethylester. Geeignete Alkohole sind beispielsweise Ethylenglykol, Diethylenglykol, Triethylenglykol, Propan-1,2-diol, Propan-1,3-diol, Dipropylenglykol, Butan-1,3-diol, Butan-1,4-diol, Neopentylglykol, 1,6-Hexandiol, 1,4-Bis(hydroxymethyl)-cyclohexan, Trimethylolpropan, Glycerin oder Pentaerythrit. Polyesterpolyole können auch durch Polymerisation von Lactonen hergestellt werden. Geeignete Lactone sind beispielsweise $\beta$-Propiolacton, $\gamma$-Butyrolacton oder $\epsilon$-Caprolacton.

Geeignete Polyesteramide werden wie Polyester hergestellt, wobei ein Teil der mehrwertigen Alkohole durch Aminoalkohole, wie beispielsweise Ethanolamin, oder Diamine, wie beispielsweise Ethylendiamin, ersetzt werden.

Geeignete Polycarbonatpolyole können durch Umsetzung von Diolen wie 1,2-Propandiol, 1,3-Propandiol, 1,4-Butandiol, Diethylenglykol mit Kohlensäureester wie Kohlensäurediphenylester, Kohlensäuredimethylester oder mit Phosgen erhalten werden.

Geeignete Polyolefinpolyole sind beispielsweise Polybutadiene mit endständigen Hydroxygruppen, die unter der Bezeichnung "Nisso-PB G-Series" von der Firma Nippon Soda vertrieben werden.

Geeignete Polysiloxanpolyole sind beispielweise Polydimethylsiloxane mit terminalen Hydroxyalkyl-Gruppen, bevorzugt Hydroxybutyl- oder Hydroxypropyl-Gruppen.

Geeignete Poly(meth)acrylatpolyole sind beispielsweise Copolymere von (Meth)Acrylsäureestern mit mindestens einer Verbindung mit mindestens einer Hydroxygruppen und mindestens einer (Meth)Acrylatgruppe. Bevorzugt handelt es sich um ein Copolymer von (Meth)Acrylsäureestern mit mindestens einer Verbindung mit nur einer Hydroxygruppen und nur einer (Meth)Acrylatgruppe.

Bevorzugt handelt es sich bei der zusätzlich mindestens einen ionischen Gruppe, der mindestens einen weiteren Verbindung, die im Sinne einer Isocyanatreaktion mono- oder difunktionell ist, um eine anionische oder kationische Gruppe.

Die anionische Gruppe ist bevorzugt eine Carboxylat-, Sulfonat-, Sulfat-, Sulfonium-, Phosphat- oder Phosphonatgruppe. Die kationische Gruppe ist bevorzugt eine Ammonium- oder Phosphoniumgruppe.

Die ionischen Gruppen und/oder die in eine ionische Gruppe umwandelbare funktionelle Gruppe können sowohl in der PU-Hauptkette als auch lateral an der PU-Hauptkette eingebaut werden.

Sterisch stabilisierende Gruppen sind polymere oder oligomere Kettenelemente, die mindestens in Wasser mindestens zum Teil löslich sind und an die Polyurethanpartikel bevorzugt kovalent angebunden sind. Sie stabilisieren die Partikel aufgrund entropischer Effekte nach dem Mechanismus der sterischen Stabilisierung (T.S. Tadros, Interfacial Phenomena and Colloid Stability, Vol. 1 Basic Principles, De Gruyter 2015, 209 ff.) in wässrigen Medien gegen Agglomeration und Koagulation. Geeignete Polymerketten sind dabei bspw. wasserlösliche Polymerketten wie Poly(oxyethylen), Poly(oxazoline), wasserlösliche Poly(2-alkyloxazoline), weitere wasserlösliche Poly(ethylenimin)-Derivate, Poly(N-vinylpyrrolidone), wasserlösliche Poly((meth)acrylate), wie beispielsweise Poly(hydroxyethyl(meth)acrylat), Poly((meth)acrylamide), wasserlösliche Polysaccharide wie beispielsweise Stärke, Pektine , Cellulose und Celluloseether wie Methylcellulose oder Hydroxy-ethylcellulose, Proteine wie beispielsweise Gelatine, teilverseiftes Polyvinylacetat oder Polyvinylalkohol.

In einer bevorzugten Ausführungsform tragen die Polymerketten keine zwischen einem pH-Wert von 1 und 9 in Wasser geladene Gruppen.

In einer weiteren Ausführungsform tragen die Polymerketten zusätzliche Gruppen, die zwischen einem pH-Wert von 1 und 9 eine Ladung tragen. Dabei kann es sich um positive oder negative Ladungsträger handeln. Geeignete chemische Gruppen sind die als ionische Gruppen beschriebenen Gruppen.

In einer spezifischen Ausführungsform ist die sterisch stabilisierende Gruppe, die auch Ladungen trägt eine Poly(Meth)acrylsäure-Kette oder eine Poly(Meth)acrylsäureCopolymer-Kette.

Geeignete relative Molekulargewichte der sterisch stabilisierenden Polymerketten liegen zwischen 200 und 100000, bevorzugt zwischen 300 und 20000 und besonders bevorzugt zwischen 400 und 4000.

Sterisch stabilisierende Gruppen sind in einer Ausführungsform Polymerketten, die über je ein Kettensegment mit dem Partikel kovalent verbunden sind. Dieses Kettensegment kann sich am Kettenende oder einer anderen Stelle der Kette befinden. Bevorzugt befindet sich das anbindende Kettensegment am Kettenende.

In einer weiteren bevorzugten Ausführungsform handelt es sich bei der zusätzlich mindestens einen ionischen und/oder sterisch stabilisierenden Gruppe, der mindestens einen weiteren Verbindung, die im Sinne einer Isocyanatreaktion mono- oder difunktionell ist, um eine sterisch stabilisierende Kette.

Die Anbindung der sterisch stabilisierenden Kette an das Partikel kann dabei auf verschiedene Weisen erfolgen. In einer bevorzugten Ausführungsform ist die Kette über eine Urethangruppe oder eine Harnstoffgruppe an das Partikel angebunden.

Typische sterisch stabilisierende Gruppen, die über ein Kettensegment mit dem Partikel verbunden sind, sind terminal monofunktionelles Methoxypoly(oxyethylen), das bspw. als $\alpha$-Methoxy,$\omega$-hydroxypoly(oxyethylen) oder $\alpha$-Methoxy,$\omega$-aminopoly(oxyethylen) bei der Partikelsynthese eingebracht wird oder Poly(methyloxazolin, das bspw. als terminal monohydroxy- oder aminofunktionalisiertes Poly(methyloxazolin) bei der Partikelsynthese eingebracht wird. Sie können auch lateral an Polyurethanketten angebunden sein.

Sterisch stabilisierende Gruppen in einer weiteren Ausführungsform sind Polymerketten, die über zwei oder mehr Kettensegmente mit dem Partikel kovalent verbunden sind. Bevorzugt sind Polymerketten, die über zwei Kettensegmente mit dem Partikel verbunden sind. In einer bevorzugten Ausführungsform befinden sich die bindenden Segmente an den Kettenenden. Dabei kann es bevorzugt sein, dass die sterisch stabilisierenden Polymerketten als Polyol A oder Polyol B bei der Synthese des Partikels eingebracht werden. In einer bevorzugten Ausführungsform sind die Ketten über eine Urethangruppe oder eine Harnstoffgruppe an das Partikel angebunden.

Ein typisches Beispiel für eine sterisch stabilisierende Gruppe, die über zwei Kettensegmente an das Partikel gebunden ist, ist Poly(oxyethylen), das bspw. als Polyethylenglycol (Polyol A oder B) oder auch $\alpha,\omega$-Diaminopoly(oxyethylen) bei der Partikelsynthese eingebracht wird. Ein anderes Beispiel ist hydroxy- oder aminotelechelisches Poly(methyloxazolin) oder Poly(ethyloxazolin).

Besonders bevorzugt handelt es sich bei der mindestens einen weiteren Verbindung, die im Sinne einer Isocyanatreaktion mono- oder difunktionell ist, um Hydrochinonmonosulfonsäure-Kaliumsalz, N-Methyldiethanolamin, PEG350, PEG600, TEGOMER® D3403 oder Ymer® N 120.

Bei dem mindestens einen Polyol B handelt es sich bevorzugt um ein Polyol B mit mindestens zwei Hydroxygruppen. Bevorzugt weist das mindestens eine Polyol B ein Molekulargewicht kleiner 500 g/mol auf. Geeignetes Polyol B kann bevorzugt aromatisch, insbesondere carbocyclisch oder heterocyclisch, oder aliphatisch, insbesondere linear, verzweigt oder cyclisch, sein. Besonders bevorzugt liegt das Polyol B aliphatisches vor. Bevorzugt handelt es sich um mindestens zweiwertige Alkohole. Geeignete Polyole B sind beispielsweise Ethylenglykol, Diethylenglykol, Triethylenglykol, Propan-1,2-diol, Propan-1,3-diol, Dipropylenglykol, Butan-1,3-diol, Butan-1,4-diol, Neopentylglykol, 1,6-Hexandiol, 1,4-Bis(hydroxymethyl)-cyclohexan, Trimethylolpropan, Glycerin und Pentaerythrit.

Bei dem Kettenverlängerer handelt es sich bevorzugt um ein Diamin mit einem Molekulargewicht von kleiner 500 g/mol. Bevorzugte Diamine sind $\alpha,\omega$-Alkylendiamine wie Ethylendiamin, 1,3-Propylendiamin, 1,4-Butylendiamin, 1,6-Hexamethylendiamin oder auch höhermolekulare primäre und sekundäre Diamine, besonders bevorzugt um Ethylendiamin. Weitere bevorzugte Ausführungsformen von Diaminen sind Diamine, deren organischer Rest, der zwischen den Aminogruppen liegt, weitere Heteroatome, insbesondere Sauerstoffatome trägt.

Bei dem Vernetzer handelt es sich bevorzugt um einen mit mindestens drei funktionellen Gruppen, wie beispielsweise Diethylentriamin.

Die Herstellung solcher Polyurethanpartikel ist u.a. beschrieben in:

Robin et al., Polym. Int. 61, 495-510, 2012,
Ramesh et al., J. Macromol. Sci. C, 38, 481-509, 1998,
Long et al., Macromol. Chem. Phys. 215, 2161-2174, 2014, und
Kim, Colloid Polym. Sci., 274, 599-611, 1996.

Die erfindungsgemäßen PU-Partikel tragen je nach ausgewählter Komponente b), c) und/oder d) Urethan-, Harnstoff-, Allophanat- und/oder Biuretgruppen als Verknüpfungsstellen. Dabei können all diese Gruppen enthalten sein oder auch nur einzelne. Es kann bevorzugt sein, PU-Partikel zu verwenden, die ausschließlich Urethangruppen als Verknüpfungsstellen aus dem Polyadditionsschritt enthalten. Es kann aus Gründen der Hydrolysestabilität auch bevorzugt sein, PU-Partikel zu verwenden, die ausschließlich oder zumindest vorwiegend Harnstoffgruppen als Verknüpfungsstellen aus dem Polyadditionsschritt enthalten.

Es ist bevorzugt, dass die Polymerpartikel keine polymerisierbaren Gruppen, insbesondere keine (Alkyl)Acrylat- oder (Alkyl)Acrylamidgruppen, aufweisen.

Säurereaktives Pulver

Geeignete säurereaktive Pulver sind dem Fachmann bekannt. Es ist bevorzugt, dass das säurereaktive Pulver ausgewählt ist aus Metalloxiden, Metallhydroxiden, Mineral Trioxid Aggregat (MTA), Hydroxylapatit, bioaktiven Gläsern, säurereaktiven Gläsern und Mischungen hiervon.

Bevorzugt sind die Metalloxide ausgewählt aus Magnesiumoxid, Calciumoxid, Strontiumoxid, Bariumoxid, Zinkoxid, Lanthanoxid, Yttriumoxid und Mischungen hiervon.

Bevorzugt sind die Methallhydroxide ausgewählt aus Magnesiumhydroxid, Calciumhydroxid, Strontiumhydroxid, Lanthanhydroxid, Yttriumhydroxid und Mischungen hiervon.

Mineral Trioxid Aggregat (MTA) ist ein Pulver bestehend aus den Hauptbestandteilen Calciumsilicat (Dicalciumsilicat $((CaO)_2 \cdot SiO_2)$ und Tricalciumsilicat $((CaO)_3 \cdot SiO_2)$), Tricalciumaluminat $((CaO)_3 \cdot Al_2O_3)$ und Calciumoxid. Als weitere Bestandteile können bevorzugt Gips $(CaSO_4 \cdot 2H_2O)$ und Bismut(III)-oxid enthalten sein.

Bioaktive Gläser sind eine Gruppe von oberflächenaktiven Gläsern, die Bioaktivität aufweisen. Sie zeichnen sich im Gegensatz zu herkömmlichen Gläsern dadurch aus, dass diese in einem wässrigen Medium löslich sind und an ihrer Oberfläche eine Hydroxylapatitschicht ausbilden. Bevorzugte bioaktive Gläser sind beispielsweise Bioglas 45S5 oder Biogläser, wie sie in der WO 2011/000866, WO 2011/161422 und WO 2014/154874 beschrieben sind.

Als säurereaktive Gläser können bevorzugt Alumosilikatgläser, bevorzugter Fluoralumosilikatgläser, noch bevorzugter Calciumfluoralumosilikatgläser und Strontiumfluoralumosilikatgläser eingesetzt werden. Außerdem sind weiter auch Gläser, enthaltend Lanthanoide bevorzugt. Geeignete Gläser sind insbesondere solche, wie sie beispielsweise in EP 0 885 854 B1, DE 3804469 C2 oder EP 1343452 B1 beschrieben sind.

Bevorzugte Bestandteile geeigneter Glaspulver sind $SiO_2$, $Al_2O_3$, $CaF_2$, $AlF_3$, $NaF$, $AlPO_4$, $CaO$, $SrO$, $SrF_2$, $P_2O_5$, $B_2O_3$, $Bi_2O_3$, $MgO$, $TiO_2$, $ZrO_2$, $GeO_2$, $La_2O_3$ oder weitere Oxide der Lanthanoiden-Reihe und $ZnO$. Geeignete Kombinationen sind beispielsweise solche, die in A.D. Wilson und J.W. Nicholson, Acid-base cements: Their biomedical and industrial applications, Cambridge Press, 1993, beschrieben sind.

Es kann bevorzugt sein, dass die Oberflächen eines Pulvers nicht mit organischen Verbindungen behandelt oder modifiziert sind. Noch bevorzugter ist, dass diese keine polymerisierbaren Gruppen, insbesondere keine (Alkyl)Acrylat- oder (Alkyl)Acrylamidgruppen, aufweisen.

Die säurereaktiven Pulver weisen bevorzugt mittlere Partikelgrößen (d50) von 0,5 bis 30 $\mu$m, weiter bevorzugt 1 bis 20 $\mu$m auf und/oder eine maximale Partikelgröße (d99) von <150 $\mu$m, bevorzugt <100 $\mu$m, weiter bevorzugt <80 $\mu$m (bspw. Laser Particle Sizer, Beckman Coulter LS 13320). Das säurereaktive Pulver umfasst bevorzugt eine erste Menge Glaspartikel mit einer mittleren Partikelgröße von 5 bis 20 $\mu$m, bevorzugter 5 bis 15 $\mu$m, und eine zweite Menge Glaspartikel mit einer mittleren Partikelgröße von 1 bis 5 $\mu$m, bevorzugter 2 bis 3 $\mu$m. Die erste und zweite Menge der Glaspartikel unterscheiden sich voneinander, beispielsweise durch eine unterschiedliche Glaszusammensetzung, einer durchgeführten Oberflächenbehandlung und/oder einer unterschiedlichen Gestalt, wie beispielsweise eine unregelmäßige oder runde Form, auf.

Bevorzugt beträgt ein Anteil von säurereaktivem Pulver im Dentalzement 20 bis 90 Gew.-%, bevorzugter 40 bis 85 Gew.-%, bezogen auf die Gesamtzusammensetzung des Dentalzements vor der Aushärtung.

Mehrprotonige Säure

Geeignete mehrprotonige Säuren sind dem Fachmann bekannt. Es ist bevorzugt, dass die mehrprotonige Säure ausgewählt ist aus Polysäuren und Phosphorsäure, besonders bevorzugt Polysäure.

Bevorzugte Polysäuren sind Homo- und Copolymere ungesättigter Carbonsäuren oder Phosphonsäuren. Noch bevorzugter sind Polyalkensäuren als Polysäuren. Besonders bevorzugt sind Polyacrylsäure (PAA), Poly(acrylsäure-co-maleinsäure), Poly(acrylsäure-co-itakonsäure), Poly(acrylsäure-co-vinylphosphonsäure) und Poly(vinylphosphonsäure). Weitere Monomere und Comonomere für geeignete Homo- und Copolymere sind in Schricker et al., J. Mat. Chem. 22, 2824-2833, 2012, beschrieben, wie beispielsweise N-Vinylcaprolactam, N-Vinylpyrrolidon, Aminosäure-modifizierte Acrylate und Acrylamide.

In einer bevorzugten Ausführungsform enthält die mehrprotonige Säure keine radikalisch polymerisierbare Gruppe, insbesondere keine (Alkyl)Acrylsäureester- oder (Alkyl)Acrylamidgruppe.

Bevorzugt beträgt ein Anteil von mehrprotoniger Säure im Dentalzement 4,9 bis 40 Gew.-%, bevorzugter 7,4 bis 25 Gew.-%, bezogen auf die Gesamtzusammensetzung des Dentalzements vor der Aushärtung.

Wasser

Bevorzugt beträgt ein Anteil von Wasser im Dentalzement 4,9 bis 40 Gew.-%, bevorzugter 7,4 bis 25 Gew.-%, bezogen auf die Gesamtzusammensetzung des Dentalzements vor der Aushärtung.

Weitere zusätzliche Bestandteile

In einer bevorzugten Ausführungsform weist der wasserhärtende Dentalzement weitere zusätzliche Bestandteile auf, ausgewählt aus der Gruppe bestehend aus Komplexierungsmitteln, anorganischen und organischen Füllstoffen, anorganischen und organischen Farbstoffen sowie Mischungen hiervon. Geeignete Komplexierungsmittel sind beispielsweise Weinsäure, Zitronensäure und/oder solche, die in Prosser et al., J. Dent. Res., 61, 1982, 1195-1198, beschrieben sind. Geeignete anorganische Füllstoffe sind beispielsweise röntgenopake nicht säurereaktive (inerte) anorganische Füllstoffe.

Gegenstand der Erfindung ist ferner ein Verfahren zum Herstellen eines wasserhärtenden Dentalzements, wobei zumindest ein säurereaktives Pulver, eine mehrprotonige Säure, Wasser und dispergierte Polymerpartikel gemischt werden.

Das erfindungsgemäße Verfahren zum Herstellen eines wasserhärtenden Dentalzements kann mit weiteren Merkmalen fortgebildet werden, die im Zusammenhang mit dem erfindungsgemäßen wasserhärtenden Dentalzement und dem erfindungsgemäßem Kit beschrieben sind.

Ein weiterer Gegenstand der Erfindung ist ein Kit zum Herstellen eines wasserhärtenden Dentalzements umfassend die Bestandteile:

a) dispergierte Polyurethan-Polymerpartikel,
b) ein säurereaktives Pulver,
c) eine mehrprotonige Säure, und
d) Wasser.

Es ist bevorzugt, dass ein Anteil dispergierter Polymerpartikel im Kit mindestens 0,005 Gew.-%, bevorzugter mindestens 0,01 Gew.-%, bezogen auf die Gesamtzusammensetzung des Kits vor der Aushärtung des Dentalzements, beträgt. Die Gewichtsangaben beziehen sich dabei auf die Polymerpartikel als solche und nicht auf die Partikeldispersion.

Weiter ist es bevorzugt, dass ein Anteil dispergierter Polymerpartikel im Dentalzement höchstens 10 Gew,-%, bevorzugter höchstens 3 Gew.-%, noch bevorzugter höchstens 1 Gew.-%, weiter bevorzugt höchstens 0,5 Gew.-%, am meisten bevorzugt höchstens 0,3 Gew.-%, bezogen auf die Gesamtzusammensetzung des Kits vor der Aushärtung des Dentalzements, beträgt.

Bevorzugter ist es ferner, dass ein Anteil dispergierter Polymerpartikel im Dentalzement 0,005 bis 5 Gew.-%, bevorzugter 0,005 bis 3 Gew.-%, noch bevorzugter 0,01 bis 1 Gew.-%, weiter bevorzugt 0,01 bis 0,5 Gew.-%, am meisten bevorzugt 0,01 bis 0,3 Gew.-%, bezogen auf die Gesamtzusammensetzung des Kits vor der Aushärtung des Dentalzements, beträgt.

Bevorzugte beträgt ein Anteil von säurereaktivem Pulver im Kit 20 bis 90 Gew.-%, bevorzugt 40 bis 85 Gew.-%, bezogen auf die Gesamtzusammensetzung des Kits vor der Aushärtung des Dentalzements.

Bevorzugt beträgt ein Anteil von mehrprotoniger Säure im Kit 4,9 bis 40 Gew.-%, bevorzugter 7,4 bis 25 Gew.-%, bezogen auf die Gesamtzusammensetzung des Kits vor der Aushärtung des Dentalzements.

Bevorzugt beträgt ein Anteil von Wasser im Kit 4,9 bis 40 Gew.-%, bevorzugter 7,4 bis 25 Gew.-%, bezogen auf die Gesamtzusammensetzung des Kits vor der Aushärtung des Dentalzements.

Der erfindungsgemäße Kit kann mit weiteren Merkmalen fortgebildet werden, die im Zusammenhang mit dem erfindungsgemäßen wasserhärtenden Dentalzement, insbesondere im Zusammenhang mit dessen weiteren und zusätzlichen Bestandteilen, beschrieben sind.

Es ist bevorzugt, dass der Kit aus mindestens zwei Komponenten besteht und die Bestandteile des Kits auf diese aufgeteilt sind. Die Bestandteile des Kits können je nach Ausführungsform bevorzugt in der ersten und/oder zweiten Komponente enthalten sein, solange die Bestandteile aus säurereaktivem Pulver, mehrprotoniger Säure und Wasser nicht nur in einer einzigen Komponente vorhanden sind. Dabei sind verschiedene Kombinationen mit jeweils verschiedenen Vorteilen verbunden, beispielsweise bezüglich der Lagerstabilität und der Anmischbarkeit. Die dispergierten Polymerpartikel können je nach Ausführungsform bevorzugt in der ersten und/oder zweiten Komponente enthalten sein. In einer bevorzugten Ausführungsform enthält die erste Komponente das säurereaktive Pulver und die zweite Komponente enthält Wasser und die dispergierten Polymerpartikel. Die mehrprotonige Säure ist je nach Ausführungsform in der ersten und/oder zweiten Komponente enthalten. Es ist erforderlich, dass zur Herstellung des wasserhärtenden Dentalzements die mindestens zwei Komponenten kurz vor der Anwendung gemischt werden.

Weiter ist es bevorzugt, dass eine erste Komponente des Kits als Pulver und eine zweite Komponente als Flüssigkeit bereitgestellt ist oder eine erste Komponente des Kits als Paste und eine zweite Komponente als Paste bereitgestellt ist.

Zudem ist es bevorzugt, dass der Kit Vorrichtungen aufweist, die zum Mischen der Komponenten geeignet sind. Bevorzugte Vorrichtungen zum Mischen sind beispielsweise Spatel und Mischunterlagen und/oder Vorrichtungen in denen die Teile des Kits vordosiert enthalten sind und/oder Vorrichtungen zum automatischen Mischen der Teile.

Bevorzugt ist ein Mischverhältnis von einer Pulver-Komponente zu einer Flüssigkeit-Komponente größer als 1:1, bevorzugter größer 2:1, noch bevorzugter größer 3:1. Dies bedeutet, dass beispielsweise 3,5 Gewichtsteile Pulver mit 1 Gewichtsteil Flüssigkeit gemischt werden.

Als geeignete Kit-Komponentensysteme sind beispielhaft die in EP 1 344 500 B1 beschriebene Kapseln sowie Kartuschensysteme zum automatischen Anmischen, wie beispielsweise die kommerziell erhältlichen Mixpac™ L- und S-Systeme, genannt.

Ein weiterer Gegenstand der Erfindung ist die Verwendung des erfindungsgemäßen wasserhärtenden Dentalzements als Füllung, noch bevorzugter als kaulasttragende Füllung.

Die Erfindung wird nun anhand vorteilhafter Ausführungsformen unter Bezugnahme auf die beigefügten Zeichnungen erläutert. Es zeigt:

Fig. 1:    Mittelwerte und Standardabweichungen für die Risszähigkeit (Spannungsintensitätsfaktor $K_{Ic}$ (in $\mathrm{MPa} \cdot \sqrt{m}$ )) der in der Tabelle 4 gezeigten Zemente 1-3.

Fig. 2:    Mittelwerte und Standardabweichungen für die Druckfestigkeit (in MPa) der in der Tabelle 4 gezeigten Zemente 1-2.

Fig. 3:    Mittelwerte und Standardabweichungen für die Biegefestigkeit (in MPa) der in der Tabelle 4 gezeigten Zemente 1-3.

Chemikalien und deren Vorbehandlung/Verwendung

| | |
|---|---|
| Polytetrahydrofuran 1000 | Merck KGaA |
| 4,4'-Diisocyanatodicyclohexylmethan (H12MDI) | Sigma Aldrich |
| Hydrochinonmonosulfonsäure-Kaliumsalz | Sigma Aldrich |
| N-Methyldiethanolamin | Sigma Aldrich |
| n-Butylmethacrylat | Sigma Aldrich |
| Kaliumperoxodisulfat | Sigma Aldrich |
| Methylmethacrylat | Evonik Performance Materials GmbH |
| Polyacrylsäure, $M_N$ = 14000 g/mol, $M_W$ = 49000 g/mol, gemahlen, d50 kleiner 50 $\mu$m | |

(fortgesetzt)

| hyperpure Wasser (GPR Rectapure) | VWR International |
|---|---|

Das Polytetrahydrofuran 1000 wurde 4 Stunden bei 0,03 mbar auf 60°C erhitzt, so dass ein getrocknetes Polytetrahydrofuran (PTHF) erhalten wurde. Das Pulver wurde unter Stickstoff gelagert.

Fluoralumosilikatglas A (FAS A):

| Zusammensetzung | Si als $SiO_2$ | 32,2 Gew.-% |
|---|---|---|
| | Al als $Al_2O_3$ | 31,6 Gew.-% |
| | Sr als $SrO$ | 24,9 Gew.-% |
| | P als $P_2O_5$ | 5,2 Gew.-% |
| | Na als $Na_2O$ | 1,7 Gew.-% |
| | F als $F^-$ | 7,2 Gew.-% |

Das Glaspulver wurde in einer Kugelmühle auf einen mittleren Partikeldurchmesser $d_{50}$ = 2,6 μm gemahlen. Das Pulver wurde 8 Stunden bei 500°C getempert.

Fluoralumosilikatglas B (FAS B):

| Zusammensetzung | $SiO_2$ | 36,00 Gew.-% |
|---|---|---|
| | $Al_2O_3$ | 22,50 Gew.-% |
| | $CaF_2$ | 21,00 Gew.-% |
| | $Na_3AlF_6$ | 9,00 Gew.-% |
| | $AlF_3$ | 6,60 Gew.-% |
| | $AlPO_4$ | 5,00 Gew.-% |

Das Glaspulver wurde in einer Kugelmühle auf einen mittleren Partikeldurchmesser $d_{50}$ = 7,7 μm gemahlen. 1 kg des Pulvers wurde anschließend in einer Lösung von 30 g $KH_2PO_4$ in 3 l dest. Wasser suspendiert und 24 Stunden bei Raumtemperatur (RT) gerührt. Anschließend wurde filtriert, mit dest. Wasser gewaschen und 6 Stunden bei 100 °C getrocknet.

Methoden

Mittlerer Partikeldurchmesser und mittleres Zetapotential der ionischen Polyurethanpartikel (PU-Partikel):

Die Parameter wurden mittels dynamischer Lichtstreuung mit dem ‚Zetasizer Nano-ZS' der Firma Malvern bestimmt. Der mittlere Partikeldurchmesser wurde als mittlerer hydrodynamischer Äquivalenzradius in Form des Z-Average gemessen. Die Partikel lagen als wässrige Dispersionen vor. Dispersionen nach Herstellung wurden 1:10 mit hyperpure Wasser verdünnt. Für die Messungen wurde Wasser als Dispergiermedium mit folgenden Parametern verwendet:

Brechungsindex 1,33,
Dielektrizitätskonstante 78,5, und
Viskosität 0,8873 cP.

Die Messungen wurden bei 25°C durchgeführt.

Partikelgröße der Fluoralumosilikatgläser:

Die Partikelgrößenverteilung und der mittlere Partikeldurchmesser ($d_{50}$-Wert) wurden mit einem Beckman Coulter Laser Particle Sizer LS130 und einem Beckman Coulter Laser Particle Sizer LS13320 bestimmt.

250 mg des gemahlenen Glases wurden mit 4 Tropfen Glycerin auf einem angerauten Uhrglas zu einer cremigen Paste angemischt. Diese Paste wurde mit 1 Tropfen Wasser mit einem Stößel vordispergiert. Anschließend wurde die Paste in 5 ml Wasser eingemischt und 5 Minuten in einem Ultraschallbad (Bandelin Sonorex RK102H) unter Eiswasserkühlung dispergiert. Die Dispersion wurde in die Messkammer des Particle-Sizers (Coulter LS130 bzw. Beckman-Coulter LS13320) eingebracht und unter Umpumpen der zu vermessenden wässrigen Dispersion vermessen.

Die Messung erfolgte in Leitungswasser. Die Auswertung erfolgte nach dem optischen Modell der Fraunhofer Beugung.

Gefriertrocknung:

Die Gefriertrocknung verdünnter Lösungen erfolgte mit dem Gefriertrockner ‚Sublimator VaCo 5‘ der Firma ZIRBUS technology GmbH.

Biegefestigkeit (BF):

Die Biegefestigkeit wurde entsprechend ISO 9917-2:2010 mit einer Vorschubgeschwindigkeit von 0,8 mm/min gemessen.

Druckfestigkeit (DF):

Die Druckfestigkeit wurde entsprechend ISO 9917-1:2010 mit einer Vorschubgeschwindigkeit von 1 mm/min gemessen.

Risszähigkeit ($K_{IC}$):

Pulver und Flüssigkeit wurden im jeweils angegebenen Anmischverhältnis innerhalb 1 Minute mittels eines Spatels auf einer Unterlage gemischt und in eine Form gefüllt mit den Dimensionen Länge L = 50 mm, Höhe H = 4 mm und Breite B = 3 mm. Nach 1 Stunde bei 37°C und >95 relativer Luftfeuchtigkeit wurde der Prüfkörper entformt und weitere 23 Stunden ± 1 Stunde in dest. Wasser bei 37°C gelagert. Die Prüfkörper wurden mit einer Niedertourensäge (Isomet der Fa. Buehler; Diamanttrennscheibe D46/54, Dicke 0,20 mm, der Fa. Boma) auf einer der 3 mm breiten Seiten gekerbt. Die Kerbtiefe betrug ca. 0,7 mm.

Die Breite und die Höhe eines Prüfkörpers wurden mittels Schieblehre gemessen. Der Prüfkörper wurde mit der Kerbe nach unten auf der 3-Punkt-Biegebruch-Biegevorrichtung (Abstand zwischen den Auflegern S = 20 mm) einer Universalprüfmaschine (Zwick Z2.5., Fa. Zwick Roell) platziert, so dass sich die Kerbe genau unter dem die Kraft übertragenden Keil befand. Die Messung der maximalen Kraft ($F_{max}$) bis zum Bruch der Prüfkörper wurde durchgeführt bei einer Vorschubgeschwindigkeit von 0,8 mm/min.

Es wurde ein Bild der Querschnittsfläche des gebrochenen Prüfkörpers unter dem Mikroskop (Leica Leitz DMRX) mit einer Digitalkamera (Leica DFC295) aufgenommen. An drei Stellen wurden die Kerbtiefen a1, a2 und a3 der Kerbe mittels Auswertesoftware (Leica Applikation Suite V3) ermittelt. Aus den drei Werten wurde der Mittelwert a gemäß Formel gebildet. Mittels der gemessenen Werte wurde die Risszähigkeit (Spannungsintensitätsfaktor $K_{IC}$) entsprechend der aufgeführten Formel berechnet.

$$a = \frac{a1+a2+a3}{3}$$

$$f\left(\frac{a}{H}\right) = \frac{3\left(\frac{a}{H}\right)^{1/2}\left[1,99 - \left(\frac{a}{H}\right)\left(1 - \left(\frac{a}{H}\right)\right)\left\{2,15 - 3,93\left(\frac{a}{H}\right) + 2,7\left(\frac{a}{H}\right)^2\right\}\right]}{2\left(1 + 2\left(\frac{a}{H}\right)\right)\left(1 - \left(\frac{a}{H}\right)\right)^{3/2}}$$

$$K_{IC} = \frac{F_{max} \cdot S \cdot 10^{-6}}{B \cdot H^{3/2}} \cdot f\left(\frac{a}{H}\right)$$

Beispiel 1

Synthese der PU-Partikel mit anionischen Gruppen:

10 g PTHF, 13,9 ml Aceton, 5,25 g H12MDI und 0,02 ml Katalysatorlösung (Dimethylzinndineodecanoat in Toluol, Masseanteil 50%) wurden 4 Stunden auf 60°C erwärmt (Rückflusskühler, Trockenrohr) und dann auf Raumtemperatur (RT) abgekühlt. Der Isocyanatgruppengehalt wurde mittels Titration bestimmt. Er betrug 2,96 Gew.-%.

Bei Raumtemperatur (ca. 23°C) wurden nun 1,65 g Hydrochinonmonosulfonsäure-Kaliumsalz (DMSO-Lösung, Masseanteil 10%) zugegeben, 4 Stunden auf 60°C und anschließend 1,5 Stunden auf 70°C erwärmt und dann auf RT abgekühlt. Der Isocyanatgruppengehalt betrug 0,43 Gew.-%.

Anschließend wurde auf 50°C erwärmt und über ca. 35 min 38,3 ml entionisiertes Wasser zugetropft. Es entstand eine milchigweiße Trübung. Am Rotationsverdampfer wurde das Aceton entfernt. Der mittlere Partikeldurchmesser der verbliebenen Dispersion betrug Z-Average = 77 nm und das mittlere Zetapotential = -31 mV.

Die Dispersion wurde mittels Dialyse aufgereinigt. Dazu wurde die ca. 20 Gew.-% Polyurethanpartikel enthaltende Dispersion mit entionisiertem Wasser verdünnt. 100 ml der verdünnten Dispersion wurden über 5 Tage gegen 8 Liter entionisiertes Wasser dialysiert (Dialyseschlauch aus regenerierter Zellulose (,Zellutrans', Carl Roth GmbH, MWCO = 6000-8000)). Während dieser Zeit wurde das Wasser viermal gewechselt. Der Feststoffgehalt der Dispersion wurde über Gefriertrocknung ermittelt und betrug 0,97 Gew.-%.

Beispiel 2

Synthese der PU-Partikel mit kationischen Gruppen:

10 g PTHF, 13,9 ml Aceton, 5,25 g H12MDI und 0,02 ml Katalysatorlösung wurden 4 Stunden auf 60°C erwärmt und dann auf RT abgekühlt. Der Isocyanatgruppengehalt betrug 3,25 Gew.-%.

Es wurden 0,956 ml N-Methyldiethanolamin zugegeben, weitere 4 Stunden auf 60°C erwärmt und wieder abgekühlt.

Anschließend wurden 1,94 ml Eisessig und 47 ml Aceton zugegeben, auf 40°C erwärmt und dann über ca. 35 min 35 ml entionisiertes Wasser zugetropft. Es entstand eine milchigweiße Trübung.

Am Rotationsverdampfer wurde das Aceton entfernt. Der mittlere Partikeldurchmesser der verbliebenen transparenten Dispersion betrug Z-Average = 35 nm und das mittlere Zetapotential 69 mV. Der Feststoffgehalt betrug 22,2 Gew.-%.

Beispiel 3

Synthese der PU-Partikel ohne ionische Gruppen:

Ansatz 1:

| | |
|---|---|
| H12MDI | 0,04 mol |
| 1,4-Butandiol | 0,005 mol |
| Terathane 650 | 0,005 mol |
| PEG600 | 0,01 mol |
| DABCO | 1 Spatelspitze |
| DBTDL | 3 Tropfen |
| LM | Aceton |

Ansatz 2:

| | |
|---|---|
| H12MDI | 0,2 mol |
| PolyTHF250 | 0,1 mol |
| PEG350 | 0,1 mol |
| DABCO | 1 Spatelspitze |
| DBTDL | 3 Tropfen |
| LM | THF |

Diisocyanat und 2 Diol-Komponenten wurden jeweils im angegeben Lösungsmittel (LM) gelöst. Weiter wurden 1,4-Diazabicyclo[2.2.2]octan (DABCO) und Dibutylzinndilaurat (DBTDL) als Katalysator zur Reaktionslösung gegeben.

Die Lösung wurde 24 Stunden gerührt, sodass eine vollständige Reaktion erhalten wurde. Die erhaltenen Präpolymere wurden unter heftigem Rühren in einen Überschuss Wasser getropft. Hierbei erfolgte instantane Partikelbildung. Die erhaltenen wässrigen Partikelsuspensionen wurden mittels Ultrafiltration dreimal mit Wasser gereinigt. Der Rückstand der Ultrafiltration wurde jeweils mit einigen Milliliter Wasser redispergiert. Die entstandenen Dispersionen wurden auf ihren Feststoffgehalt gravimetrisch untersucht und anschließend zur Herstellung der Flüssigkeiten für die Glasionomerzemente verwendet. Mit Hilfe des Particle-Sizers wurde die Größe der erhaltenen Partikel bestimmt:

Ansatz 1

Feststoffgehalt der Dispersion: 18,6 Gew.%
Größe der Partikel: 300 nm

Ansatz 2

Feststoffgehalt der Dispersion: 16,3 Gew.%
Größe der Partikel: 165 nm

Beispiel 4

Herstellung der Kitbestandteile:

Pulver:

18,2 Teile Polyacrylsäure (PAS) und 81,8 Teile FAS B wurden miteinander vermischt.

Flüssigkeit:

Die erfindungsgemäßen Flüssigkeiten wurden durch Mischen von entionisiertem Wasser und den im Beispiel 3 erhaltenen Dispersionen hergestellt.

Herstellung von wasserhärtenden Dentalzementen:

Pulver und Flüssigkeit wurden in den in den Tabellen 1 und 2 angegebenen Gewichtsverhältnissen vermischt, Prüfkörper geformt und die Risszähigkeit bestimmt. Angegeben sind die Risszähigkeit $K_{1c}$ und die Standardabweichung (SD).

Tabelle 1: Herstellung der wasserhärtenden Zemente unter Verwendung der Dispersion (Disp) aus Ansatz 1, Beispiel 3 (300 nm) .

| Pulver [g] | Flüssig keit [g] | Zusammensetzung Flüssigkeit m(H$_2$O): m(Disp) [g/g] | PU-Partikel in der Mischung [Gew.-%] | $K_{1c}$ [MPa/m$^{1/2}$] | SD | Erhöhung [%] |
|---|---|---|---|---|---|---|
| 5,40 | 1,00 | 1,00:0 | 0 | 0,365 | 0,038 | - |
| 1,40 | 0,268 | 1,44:0,276 | 0,48 | 0,430 | 0,075 | 18 |
| 5,40 | 1,19 | 0:1,00 | 3,35 | 0,461 | 0,039 | 26 |

Tabelle 2: Herstellung der wasserhärtenden Dentalzemente unter Verwendung der Dispersion aus Ansatz 2, Beispiel 3 (165 nm).

| Pulver [g] | Flüssi gkeit [g] | Zusammensetzung Flüssigkeit m $(H_2O)$: m(Disp) [g/g] | PU-Partikel Mischung [Gew.-%] | $K_{1c}$ [MPa/m$^{1/2}$] | SD | Erhöhung [%] |
|---|---|---|---|---|---|---|
| 5,40 | 1,00 | 1,00:0 | 0 | 0,365 | 0,038 | - |
| 5,40 | 1,03 | 3,78:3,78 | 1,31 | 0,465 | 0,037 | 27 |

Beispiel 5

Herstellung der Kitbestandteile (Pulver und Flüssigkeit)

Pulver:

47,5 Teile FAS A, 31,7 Teile FAS B und 20,8 Teile PAA wurden miteinander vermischt.

Flüssigkeit:

Die Flüssigkeiten wurden durch Mischen von entionisiertem Wasser, Weinsäure und den in den Beispielen 1 und 2 erhalten Dispersionen hergestellt. Die Zusammensetzung der Flüssigkeiten ist in Tabelle 3 angegeben.

Tabelle 3: Zusammensetzung der Flüssigkeiten.

| | Flüssigkeit 1 (L1) | | Flüssigkeit 2 (L2) | | Flüssigkeit 3 (L3) | |
|---|---|---|---|---|---|---|
| | [g] | [%] | [g] | [%] | [g] | [%] |
| Wasser | 23,78 | 94,97 | 5,366 | 50,95 | 18,435 | 92,11 |
| Dispersion aus Beispiel 1 | - | - | 4,640 | 44,06 | - | - |
| Dispersion aus Beispiel 2 | - | - | - | - | 0,575 | 2,87 |
| Weinsäure | 1,26 | 5,03 | 0,526 | 4,99 | 1,004 | 5,02 |
| Summe | 25,04 | 100,00 | 10,532 | 100,00 | 20,014 | 100,00 |
| PU-Gehalt | - | - | 0,045 | 0,427 | 0,128 | 0,640 |

Beispiel 6

Herstellung von wasserhärtenden Dentalzementen:

Je 2,4 g Pulver und 0,38 g Flüssigkeit wurden innerhalb einer Minute mittels Spatel auf einer Unterlage vermischt und Prüfkörper geformt. Von den abgebundenen Prüfkörpern wurden wie beschrieben Druckfestigkeit (DF), Biegefestigkeit (BF) sowie Risszähigkeit ($K_{IC}$) bestimmt.

Tabelle 4: Eigenschaften der wasserhärtenden Dentalzemente.

| | Zement 1 | Zement 2 | Zement 3 |
|---|---|---|---|
| Pulver [g] | 2,4 | 2,4 | 2,4 |
| L1 [g] | 0,38 | - | - |
| L2 [g] | - | 0,38 | - |
| L3 [g] | - | - | 0,38 |
| DF (MPa, 24 h) | 255 ± 6 | 245 ± 17 | - |
| BF (MPa, 24 h) | 39,8 ± 7,1 | 44,8 ± 4,8 | 41,0 ± 7,7 |

(fortgesetzt)

|  | Zement 1 | Zement 2 | Zement 3 |
|---|---|---|---|
| $K_{IC}$ ( $MPa\sqrt{m}$ , 24 h) | 0,75 ± 0,03 | 0,82 ± 0,05 | 0,84 ± 0,07 |

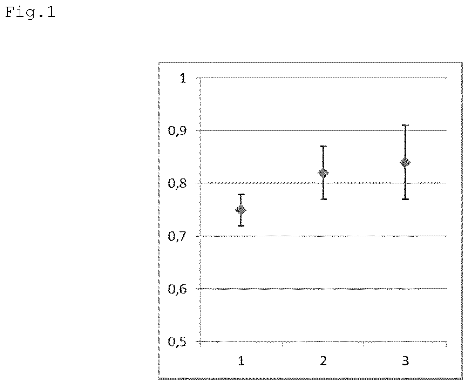

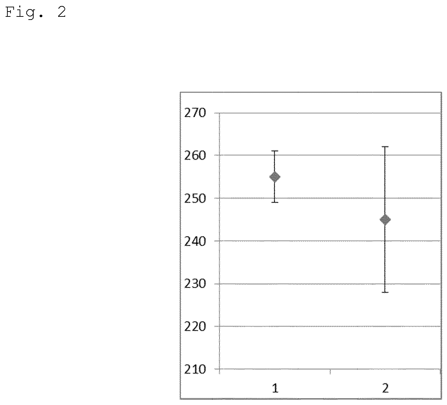

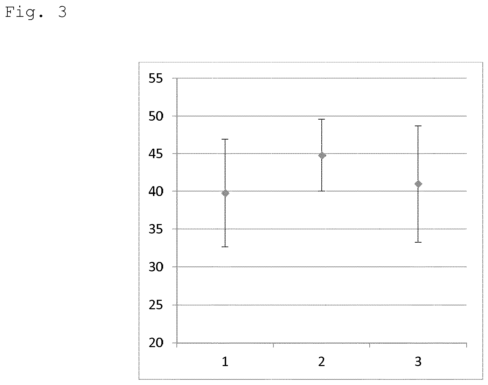

Die Masseanteile ionischer PU-Partikel an den wasserhärtenden Dentalzementen betrugen 0,06 bzw. 0,09 Gew.-%. Man erkennt, dass der Zusatz geringer Mengen dieser Polyurethanpartikel eine deutliche Erhöhung der Risszähigkeit (Spannungsintensitäts-faktor $K_{IC}$) der Zemente bewirkte (vgl. Fig. 1), während der Zusatz keinen Einfluss auf Druckfestigkeit und Biegefestigkeit hatte (vgl. Fig. 2 und 3).

Beispiel 7 - Synthese PMMA-Partikel

In einen 250 ml-Dreihalskolben ausgestattet mit einem KPG-Rührer und zwei Septen wurden 150 ml Reinstwasser vorgelegt. Der Inhalt wurde unter Stickstoffbegasung auf 90 °C erhitzt. Nach 45 min wurde der Stickstoffstrom abgestellt und es wurden 15 ml (141 mmol) Methylmethacrylat durch das Septum addiert. Um die Polymerisation zu starten, wurden nach weiteren 30 min bei 90°C 5 ml (1,8 mmol) einer 10%igen wässrigen Kaliumperoxodisulfat-Lösung als Initiator addiert. Diese Lösung wurde vorher ebenfalls für 10 min bei 90 °C mit Stickstoff begast. Die Reaktionslösung wurde mit dem KPG-Rührer bei 400 U/min gerührt. Zur Überwachung der Reaktion wurden alle 30 min je 0,1 ml Reaktionslösung durch das Septum entnommen und auf einem Glasträger an der Luft getrocknet. Wenn sich die Reflektionsfarbe des getrockneten Films nicht mehr änderte, wurde noch 30 min bei 90 °C gerührt. Um die Reaktion zu beenden, wurde das Septum entfernt, und die Suspension noch etwa 20 min an der Luft gerührt.

Die Reaktionslösung wurde zur Aufreinigung warm abfiltriert, um grobe Verunreinigungen abzutrennen. Das Filtrat wurde anschließend zentrifugiert. Zu Beginn wurde mindestens zweimal 5 bis 10 min bei 4000 U min-1 zentrifugiert, um farblosen Bodensatz abzutrennen. Danach wurde 30 bis 90 min zentrifugiert, bis sich eine klare Lösung über dem schillernden Bodensatz gebildet hat. Die flüssige Phase wurde dekantiert und der Bodensatz erneut in 60 ml destilliertem Wasser redispergiert. Dieser Vorgang wurde drei bis viermal wiederholt, um das Polymer vollständig von niedermolekularen Reaktionsrückständen zu reinigen.

Die Aufbewahrung erfolgte als 5 bis 20%ige wässrige Suspension.

Die Größe der erhaltenen Partikel wurde mit Hilfe eines Particlesizers der Firma Beckmann-Coulter mit einer mittleren Partikelgröße von 342nm bestimmt.

Beispiel 8 - Synthese PMMA-co-n-ButylMA-Partikel (80:20)

Die Synthese erfolgte analog Beispiel 7 - Synthese der PMMA-Partikel. Es wurden jedoch 12 ml (113 mmol) Methylmethacrylat sowie 4,5 ml (28 mmol) n-Butylmethacrylat addiert.

Die Größe der erhaltenen Partikel wurde mit einer mittleren Partikelgröße von 323nm bestimmt.

Beispiel 9 - Synthese PMMA-co-n-ButylMA-Partikel (60:40)

Die Synthese erfolgte analog Beispiel 7 - Synthese der PMMA-Partikel. Es wurden jedoch 9 ml (84,6 mmol) Methylmethacrylat sowie 9 ml (56 mmol) n-Butylmethacrylat addiert.

Die Größe der erhaltenen Partikel wurde mit einer mittleren Partikelgröße von 345 nm bestimmt.

Beispiel 10 - Synthese PMMA-co-n-ButylMA-Partikel (40:60)

Die Synthese erfolgte analog Beispiel 7 - Synthese der PMMA-Partikel. Es wurden jedoch 6 ml (56 mmol) Methylmethacrylat sowie 13,6 ml (85 mmol) n-Butylmethacrylat addiert.

Die Größe der erhaltenen Partikel wurde mit einer mittleren Partikelgröße von 332 nm bestimmt.

Beispiel 11 - Synthese Kern-Schale-Partikel (PnButylMA-PMMA)

Die Synthese erfolgte zunächst analog Beispiel 7 - Synthese der PMMA-Partikel. Es wurden jedoch zunächst 13,6 ml (85 mmol) undestilliertes n-Butylmethylmethacrylat addiert.

Wenn sich die Reflektionsfarbe des getrockneten Films nicht mehr änderte, wurden durch das Septum 6 ml (56 mmol) Methylmethacrylat addiert und die Reaktion nun wieder analog Beispiel 7 weitergeführt.

Die Größe der erhaltenen Partikel wurde mit einer mittleren Partikelgröße von 380 nm bestimmt.

Beispiel 12

Kitbestandteile

Das Pulver bestand aus einer homogenen Mischung aus Fluoralumosilikatglas B (FAS B) und Polyacrylsäure im Verhältnis 4,51:1.

Das Liquid (Flüssigkeit) bestand aus vollentsalztem Wasser für das Referenzsystem oder einer wässrigen Partikeldispersion für einen partikelverstärkten Glasionomerzement. Die Partikeldispersionen wurden jeweils durch Redispergieren einer entsprechenden Menge Polymerpartikel in vollentsalztem Wasser hergestellt. Die einzelnen Partikelgehalte der wässrigen Partikeldispersionen sind in Tabelle 5 angegeben.

Beispiel 13

Herstellung von Glasionomerzementen

Die Glasionomerzemente wurden mittels Spatel auf einer Unterlage aus den Kitbestandteilen angemischt. Die einzelnen Anmischverhältnisse sind in Tabelle 5 angegeben.

Tabelle 5

| Polymerpartikel | Pulver [g] | Liquid [g] | Polymerpartikel im Liquid [g/g] | Polymerpartikel im Zement [Gew.-%] | K1c [MPa/m$^{1/2}$] | SD | Steigerung K1c [%] |
|---|---|---|---|---|---|---|---|
| Referenz | 1,4 | 0,26 | 0 | 0 | 0,365 | 0,037 | - |
| Beispiel 7 | 1,4 | 0,264 | 0,015 | 0,25 | 0,402 | 0,026 | 10,06 |
| | 1,4 | 0,268 | 0,03 | 0,48 | 0,412 | 0,045 | 12,99 |
| | 1,4 | 0,273 | 0,05 | 0,75 | 0,406 | 0,042 | 10,06 |
| Beispiel 8 | 1,4 | 0,268 | 0,03 | 0,48 | 0,388 | 0,049 | 6,29 |
| Beispiel 9 | 1,4 | 0,268 | 0,03 | 0,48 | 0,400 | 0,041 | 9,64 |
| Beispiel 10 | 1,4 | 0,268 | 0,03 | 0,5 | 0,425 | 0,025 | 16,35 |
| Beispiel 11 | 1,4 | 0,264 | 0,015 | 0,25 | 0,400 | 0,044 | 9,64 |
| | 1,4 | 0,268 | 0,03 | 0,5 | 0,402 | 0,033 | 10,27 |
| | 1,4 | 0,277 | 0,065 | 1 | 0,410 | 0,025 | 12,37 |

Durch den Zusatz von 0,25 bis 0,75 Gew.-% der Polymerpartikel aus den Beispielen 7 bis 11 in wässriger Dispersion wurde die Risszähigkeit des Glasionomerzements (Referenz) deutlich verbessert.

**Patentansprüche**

1. Wasserhärtender Dentalzement umfassend ein säurereaktives Pulver, eine mehrprotonige Säure, Wasser und dispergierte Polymerpartikel, dadurch charakterisiert, dass die Polymerpartikel Polyurethanpartikel sind.

2. Wasserhärtender Dentalzement nach Anspruch 1, **dadurch gekennzeichnet, dass** der wasserhärtende Dentalzement ein Glasionomerzement, bevorzugter ein klassischer Glasionomerzement ist.

3. Wasserhärtender Dentalzement nach Anspruch 1 oder 2, **dadurch gekennzeichnet, dass** eine mittlere Partikelgröße der Polymerpartikel kleiner als etwa 1 μm, bevorzugter zwischen 5 bis 500 nm, noch bevorzugter zwischen 5 bis 100 nm ist, bestimmt durch dynamische Lichtstreuung in wässriger Dispersion (in Wasser).

4. Wasserhärtender Dentalzement nach einem der Ansprüche 1 bis 3, **dadurch gekennzeichnet, dass** die Polymerpartikel vor dem Aushärten des wasserhärtenden Zements in wässriger Lösung dispergiert sind.

**5.** Wasserhärtender Dentalzement nach einem der Ansprüche 1 bis 4, **dadurch gekennzeichnet, dass** ein Anteil dispergierter Polymerpartikel im Zement 0,005 bis 10 Gew.-%, bevorzugter 0,005 bis 3 Gew.-%, noch bevorzugter 0,01 bis 1 Gew.-%, weiter bevorzugt 0,01 bis 0,5 Gew.-%, am meisten bevorzugt 0,01 bis 0,3 Gew.-%, bezogen auf die Gesamtzusammensetzung des Zements vor der Aushärtung, beträgt.

**6.** Wasserhärtender Dentalzement nach einem der Ansprüche 1 bis 5, dass er bezogen auf die Gesamtzusammensetzung des Zements vor der Aushärtung einen oder mehrere der nachfolgend genannten Bestandteile in den nachfolgend genannten Mengenanteilen umfasst:

- 20 bis 90 Gew.-%, bevorzugt 40 bis 85 Gew.-% säurereaktives Pulver,
- 4,9 bis 40 Gew.-%, bevorzugt 7,4 bis 25 Gew.-% mehrprotonige Säure, und/oder
- 4,9 bis 40 Gew.-%, bevorzugt 7,4 bis 25 Gew.-% Wasser.

**7.** Wasserhärtender Dentalzement nach einem der Ansprüche 1 und 3 bis 6, **dadurch gekennzeichnet, dass** das säurereaktive Pulver ausgewählt ist aus Metalloxiden, Metallhydroxiden, Mineral Trioxid Aggregat, Hydroxylapatit, bioaktiven Gläsern, insbesondere säurereaktiven Gläsern und Mischungen hiervon.

**8.** Wasserhärtender Dentalzement nach einem der Ansprüche 1 bis 7, **dadurch gekennzeichnet, dass** das säurereaktive Pulver ausgewählt ist aus einer ersten Menge Glaspartikel mit einer wie in der Beschreibung definierten mittleren Partikelgröße von 5 bis 20 $\mu$m, bevorzugter 5 bis 15 $\mu$m, und eine zweite Menge Glaspartikel mit einer mittleren Partikelgröße von 1 bis 5 $\mu$m, bevorzugter 2 bis 3 $\mu$m.

**9.** Wasserhärtender Dentalzement nach einem der Ansprüche 1 bis 8, **dadurch gekennzeichnet, dass** die mehrprotonige Säure ausgewählt ist aus Polysäuren und Phosphorsäure.

**10.** Wasserhärtender Dentalzement nach einem der Ansprüche 1 bis 9, **dadurch gekennzeichnet, dass** der wasserhärtende Dentalzement weitere zusätzliche Bestandteile aufweist, ausgewählt aus der Gruppe bestehend aus Komplexierungsmitteln, anorganischen und organischen Füllstoffen, anorganischen und organischen Farbstoffen sowie Mischungen hiervon.

**11.** Verfahren zum Herstellen eines wasserhärtenden Dentalzements, **dadurch gekennzeichnet, dass** zumindest ein säurereaktives Pulver, eine mehrprotonige Säure, Wasser und dispergierte Polymerpartikel gemischt werden, wobei die Polymerpartikel Polyurethanpartikel sind.

**12.** Kit zur Herstellung eines wasserhärtenden Dentalzements umfassend die Bestandteile:

a) dispergierte Polyurethan-Polymerpartikel,
b) ein säurereaktives Pulver,
c) eine mehrprotonige Säure, und
d) Wasser,

wobei er bezogen auf die Gesamtzusammensetzung des Kits vor der Aushärtung des Dentalzements vorzugsweise einen oder mehrere der nachfolgend genannten Bestandteile in den nachfolgend genannten Mengenanteilen umfasst:

- 0,005 bis 5 Gew.-%, bevorzugt 0,005 bis 3 Gew.-%, bevorzugter 0,01 bis 1 Gew.-%, noch bevorzugter 0,01 bis 0,5 Gew.-%, am meisten bevorzugt 0,01 bis 0,3 Gew.-% dispergierte Polymerpartikel,
- 20 bis 90 Gew.-%, bevorzugt 40 bis 85 Gew.-% säurereaktives Pulver,
- 4,9 bis 40 Gew.-%, bevorzugt 7,4 bis 25 Gew.-% mehrprotonige Säure,
- 4,9 bis 40 Gew.-%, bevorzugt 7,4 bis 25 Gew.-% Wasser.

**13.** Kit nach Anspruch 12, **dadurch gekennzeichnet, dass** der Kit aus mindestens zwei Komponenten besteht und die Bestandteile des Kits auf diese aufgeteilt sind.

**14.** Wasserhärtender Dentalzement nach einem der Ansprüche 1 bis 10 zur Verwendung als Füllung oder als Befestigungszement.

**Claims**

1.  Water-hardening dental cement comprising an acid-reactive powder, a polyprotic acid, water and dispersed polymer particles, **characterized in that** the polymer particles are polyurethane particles.

2.  Water-hardening dental cement according to Claim 1, **characterized in that** the water-hardening dental cement is a glass ionomer cement, more preferably a conventional glass ionomer cement.

3.  Water-hardening dental cement according to Claim 1 or 2, **characterized in that** an average particle size of the polymer particles is less than about 1 $\mu$m, more preferably between 5 to 500 nm, yet more preferably between 5 to 100 nm, determined by dynamic light scattering in aqueous dispersion (in water) .

4.  Water-hardening dental cement according to any of Claims 1 to 3, **characterized in that** the polymer particles have been dispersed in aqueous solution prior to the hardening of the water-hardening cement.

5.  Water-hardening dental cement according to any of Claims 1 to 4, **characterized in that** a proportion of dispersed polymer particles in the cement is 0.005% to 10% by weight, more preferably 0.005% to 3% by weight, yet more preferably 0.01% to 1% by weight, further preferably 0.01% to 0.5% by weight, most preferably 0.01% to 0.3% by weight, based on the overall composition of the cement prior to hardening.

6.  Water-hardening dental cement according to any of Claims 1 to 5, **characterized in that** it comprises, based on the overall composition of the cement prior to hardening, one or more of the constituents mentioned hereinbelow in the quantitative proportions mentioned hereinbelow:

    - 20% to 90% by weight, preferably 40% to 85% by weight of acid-reactive powder,
    - 4.9% to 40% by weight, preferably 7.4% to 25% by weight of polyprotic acid, and/or
    - 4.9% to 40% by weight, preferably 7.4% to 25% by weight of water.

7.  Water-hardening dental cement according to any of Claims 1 and 3 to 6, **characterized in that** the acid-reactive powder is selected from metal oxides, metal hydroxides, mineral trioxide aggregate, hydroxyapatite, bioactive glasses, especially acid-reactive glasses and mixtures thereof.

8.  Water-hardening dental cement according to any of Claims 1 to 7, **characterized in that** the acid-reactive powder is selected from a first quantity of glass particles having an average particle size as defined in the description of from 5 to 20 $\mu$m, more preferably 5 to 15 $\mu$m, and a second quantity of glass particles having an average particle size of from 1 to 5 $\mu$m, more preferably 2 to 3 $\mu$m.

9.  Water-hardening dental cement according to any of Claims 1 to 8, **characterized in that** the polyprotic acid is selected from polyacids and phosphoric acid.

10. Water-hardening dental cement according to any of Claims 1 to 9, **characterized in that** the water-hardening dental cement includes further additional constituents selected from the group consisting of complexing agents, inorganic and organic fillers, inorganic and organic colorants and mixtures thereof.

11. Method for producing a water-hardening dental cement, **characterized in that** at least an acid-reactive powder, a polyprotic acid, water and dispersed polymer particles are mixed, wherein the polymer particles are polyurethane particles.

12. Kit for producing a water-hardening dental cement comprising the constituents:

    a) dispersed polyurethane polymer particles,
    b) an acid-reactive powder,
    c) a polyprotic acid, and
    d) water,

    wherein, based on the overall composition of the kit prior to hardening of the dental cement, it preferably comprises one or more of the constituents mentioned hereinbelow in the quantitative proportions mentioned hereinbelow:

- 0.005% to 5% by weight, preferably 0.005% to 3% by weight, more preferably 0.01% to 1% by weight, yet more preferably 0.01% to 0.5% by weight, most preferably 0.01% to 0.3% by weight of dispersed polymer particles,
- 20% to 90% by weight, preferably 40% to 85% by weight of acid-reactive powder,
- 4.9% to 40% by weight, preferably 7.4% to 25% by weight of polyprotic acid,
- 4.9% to 40% by weight, preferably 7.4% to 25% by weight of water.

13. Kit according to Claim 12, **characterized in that** the kit is composed of at least two components and the constituents of the kit are divided between these components.

14. Water-hardening dental cement according to any of Claims 1 to 10, for use as a filling or as a luting cement.

## Revendications

1. Ciment dentaire durcissant à l'eau, comprenant une poudre active par un acide, un acide à plusieurs protons, de l'eau et des particules polymères dispersées, **caractérisé en ce que** les particules polymères sont des particules de polyuréthane.

2. Ciment dentaire durcissant à l'eau selon la revendication 1, **caractérisé en ce que** le ciment dentaire durcissant à l'eau est un ciment de verre ionomère, de préférence un ciment de verre ionomère classique.

3. Ciment dentaire durcissant à l'eau selon la revendication 1 ou 2, **caractérisé en ce qu'**une grosseur moyenne de particule des particules polymères est inférieure à environ 1 $\mu$m, plus préférablement entre 5 à 500 nm, encore plus préférablement entre 5 à 100 nm, déterminée par dispersion dynamique de la lumière dans une dispersion aqueuse (dans l'eau).

4. Ciment dentaire durcissant à l'eau selon l'une des revendications 1 à 3, **caractérisé en ce que** les particules polymères sont dispersées dans une solution aqueuse avant le durcissement du ciment durcissant à l'eau.

5. Ciment dentaire durcissant à l'eau selon l'une des revendications 1 à 4, **caractérisé en ce qu'**une proportion de particules polymères dispersées dans le ciment représente 0,005 à 10% en poids, plus préférablement 0,005 à 3% en poids, encore plus préférablement 0,01 à 1% en poids, plus préférablement 0,01 à 0,5% en poids, le plus préférablement 0,01 à 0,3% en poids, par rapport à la composition totale du ciment avant le durcissement.

6. Ciment dentaire durcissant à l'eau selon l'une des revendications 1 à 5, **caractérisé en ce qu'**il comprend, par rapport à la composition totale du ciment avant le durcissement, un ou plusieurs des constituants mentionnés ci-après dans les proportions de quantités mentionnées ci-après :

   - 20 à 90% en poids, de préférence 40 à 85% en poids de poudre active par un acide,
   - 4,9 à 40% en poids, de préférence 7,4 à 25% en poids d'acide à plusieurs protons et/ou
   - 4,9 à 40% en poids, de préférence 7,4 à 25% en poids d'eau.

7. Ciment dentaire durcissant à l'eau selon l'une des revendications 1 et 3 à 6, **caractérisé en ce que** la poudre active par un acide est choisie parmi les oxydes métalliques, les hydroxydes métalliques, un agrégat minéral de trioxyde, l'hydroxylapatite, les verres bioactifs, en particulier les verres actifs par un acide et leurs mélanges.

8. Ciment dentaire durcissant à l'eau selon l'une des revendications 1 à 7, **caractérisé en ce que** la poudre active par un acide est choisie parmi une première quantité de particules de verre présentant une grosseur moyenne de particule telle que définie dans la description de 5 à 20 $\mu$m, plus préférablement de 5 à 15 $\mu$m, et une deuxième quantité de particules de verre présentant une grosseur moyenne de particule de 1 à 5 $\mu$m, plus préférablement de 2 à 3 pm.

9. Ciment dentaire durcissant à l'eau selon l'une des revendications 1 à 8, **caractérisé en ce que** l'acide à plusieurs protons est choisi parmi les polyacides et l'acide phosphorique.

10. Ciment dentaire durcissant à l'eau selon l'une des revendications 1 à 9, **caractérisé en ce que** le ciment dentaire durcissant à l'eau présente d'autres constituants supplémentaires, choisis dans le groupe constitué par les agents de complexation, les charges inorganiques et organiques, les colorants inorganiques et organiques ainsi que leurs mélanges.

11. Procédé pour la préparation d'un ciment dentaire durcissant à l'eau, **caractérisé en ce qu'**on mélange au moins une poudre active par un acide, un acide à plusieurs protons, de l'eau et des particules polymères dispersées, les particules polymères étant des particules de polyuréthane.

12. Kit pour la préparation d'un ciment dentaire durcissant à l'eau, comprenant les constituants :

      a) particules polymères dispersées de polyuréthane,
      b) une poudre active par un acide,
      c) un acide à plusieurs protons et
      d) de l'eau,

le kit comprenant, par rapport à la composition totale du kit avant le durcissement du ciment dentaire, de préférence un ou plusieurs des constituants mentionnés ci-après dans les proportions de quantités mentionnées ci-après :

      - 0,005 à 5% en poids, de préférence 0,005 à 3% en poids, plus préférablement 0,01 à 1% en poids, encore plus préférablement 0,01 à 0,5% en poids, le plus préférablement 0,01 à 0,3% en poids de particules polymères dispersées,
      - 20 à 90% en poids, de préférence 40 à 85% en poids de poudre active par un acide,
      - 4,9 à 40% en poids, de préférence 7,4 à 25% en poids d'acide à plusieurs protons,
      - 4,9 à 40% en poids, de préférence 7,4 à 25% en poids d'eau.

13. Kit selon la revendication 12, **caractérisé en ce que** le kit est constitué par au moins deux composants et les constituants du kit sont répartis parmi ceux-ci.

14. Ciment dentaire durcissant à l'eau selon l'une des revendications 1 à 10 destiné à être utilisé comme obturation ou ciment de fixation.

Fig.1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 2001354509 B
- US 6860932 B
- WO 2017083039 A1
- EP 2316407 A1
- WO 2011000866 A
- WO 2011161422 A
- WO 2014154874 A
- EP 0885854 B1
- DE 3804469 C2
- EP 1343452 B1
- EP 1344500 B1

**In der Beschreibung aufgeführte Nicht-Patentliteratur**

- Interfacial Phenomena and Colloid Stability. **T.S. TADROS.** Basic Principles. De Gruyter, 2015, vol. 1, 209
- Chemische Struktur und Synthesen. **H.G. ELIAS.** Makromoleküle. Wiley VCH, vol. 1
- **K. LANDFESTER ; F. TIARKS ; H.-P. HENTZE ; M. ANTONIETTI.** *Macromol. Chem. Phys.,* 2000, vol. 201, 1-5
- **K. LANDFESTER.** *Macromol. Rapid Commun.,* 2001, vol. 22, 896
- The Polyurethanes Book. John Wiley & Sons, Ltd, 2002
- Basic Principles. **T.S. TADROS.** Interfacial Phenomena and Colloid Stability. De Gruyter, 2015, vol. 1, 209
- **ROBIN et al.** *Polym. Int.,* 2012, vol. 61, 495-510
- **RAMESH et al.** *J. Macromol. Sci. C,* 1998, vol. 38, 481-509
- **LONG et al.** *Macromol. Chem. Phys.,* 2014, vol. 215, 2161-2174
- **KIM.** *Colloid Polym. Sci.,* 1996, vol. 274, 599-611
- **A.D. WILSON ; J.W. NICHOLSON.** Acid-base cements: Their biomedical and industrial applications. Cambridge Press, 1993
- **SCHRICKER et al.** *J. Mat. Chem.,* 2012, vol. 22, 2824-2833
- **PROSSER et al.** *J. Dent. Res.,* 1982, vol. 61, 1195-1198